(19) Europäisches Patentamt
European Patent Office
Office européen des brevets

(11) Publication number: **0 115 821 B1**

(12) **EUROPEAN PATENT SPECIFICATION**

(45) Date of publication of patent specification: **03.09.86**

(51) Int. Cl.$^4$: **C 07 D 493/10**

(21) Application number: **84100784.2**

(22) Date of filing: **25.01.84**

# (54) Fluoran compounds.

(30) Priority: **28.01.83 JP 11323/83**

(43) Date of publication of application:
**15.08.84 Bulletin 84/33**

(45) Publication of the grant of the patent:
**03.09.86 Bulletin 86/36**

(84) Designated Contracting States:
**DE FR GB IT**

(56) References cited:
**DE-A-2 834 711**
**DE-A-3 233 224**
**US-A-3 998 846**
**US-A-4 302 393**
**US-A-4 330 473**

**CHEMICAL ABSTRACTS, vol. 95, no. 2, July 13, 1981, Columbus, Ohio, USA SHIRAKAWA, TAKASHI; MIYAZAWA, YOSHIHIDE "Sensitization of free-radical sensitive materials by optical development. Relation between optical development characteristics and the molecular structure of color former" page 602, column 1, abstract-no. 15 892c**

(73) Proprietor: **HODOGAYA CHEMICAL CO., LTD.**
**4-2, Toranomon 1 Chome Minato-ku**
**Tokyo (JP)**

(72) Inventor: **Gonda, Michihiro**
**Hodogaya Chemical Co. Ltd. Chuo Kenkyusho, 2-30**
**Ohji 6-chome Kita-ku Tokyo (JP)**
Inventor: **Takeuchi, Shuichi**
**Hodogaya Chemical Co. Ltd. Chuo Kenkyusho, 2-30**
**Ohji 6-chome Kita-ku Tokyo (JP)**
Inventor: **Kanasugi, Mikiko**
**Hodogaya Chemical Co. Ltd. Chuo Kenkyusho, 2-30**
**Ohji 6-chome Kita-ku Tokyo (JP)**

(74) Representative: **Wächtershäuser, Günter, Dr.**
**Tal 29**
**D-8000 München 2 (DE)**

**EP 0 115 821 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel fluoran compounds. More particularly, the present invention relates to 2-(chloro-substituted benzylamino)-6-dibutylamino-fluoran compounds useful as a coloring material for heat or pressure sensitive record sheets.

As a typical conventional fluoran compound, (A—1) 2-benzylamino-6-diethylamino-fluoran represented by the formula:

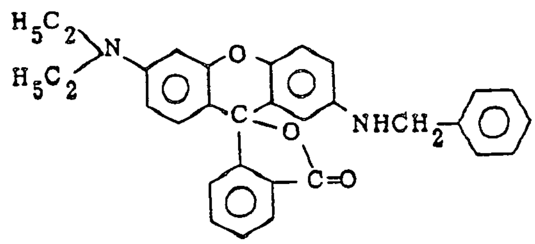

is well known (Japanese Examined Patent Publication No. 4614/1971), which undergoes color-development when brought in contact with an acidic substance. Also known are (A—2) 2-(2-chlorobenzyl-amino)-6-diethylamino-fluoran represented by the formula

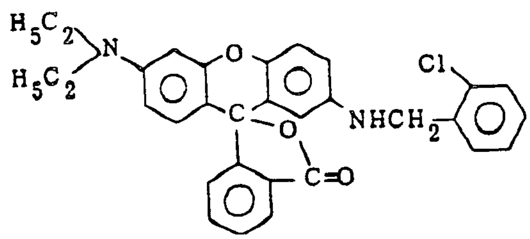

and (A—3) 2-(4-chlorobenzylamino)-6-diethylamino-fluoran represented by the formula:

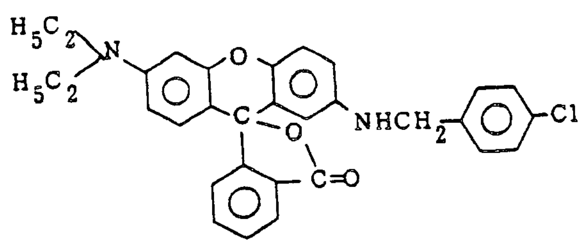

(U.S. Patent No. 3,825,561).

The inventors have found that novel fluoran compounds which are similar to the conventional fluoran compounds but have an N,N-dibutylamino group at the 6-position instead of the N,N-diethylamino group and a chloro-substituted benzylamino group at the 2-position, have superior properties as a coloring material for heat or pressure sensitive record sheets.

Namely, the present invention provides 2-(chloro-substituted benzylamino)-6-dibutylamino-fluoran compounds represented by the general formula I:

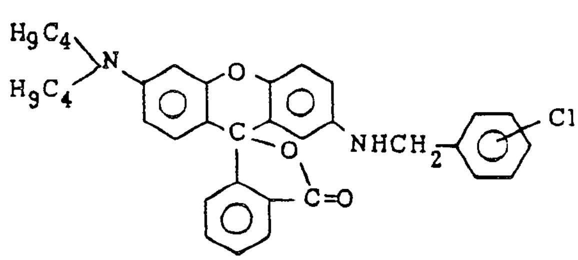

(I)

The fluoran compounds of the formula I are novel compounds, which are substantially colorless solid and which, when brought in contact with an acidic substance, i.e. an electron acceptor such as an organic acid, an acid clay, an activated acid clay, a phenol resin, a metal salt of an aromatic carboxylic acid, or bisphenol A, readily form coloring agents having a deep greenish black color. The coloring agents thus developed, have superior properties useful for a coloring material for heat or pressure sensitive record sheets.

As compared with the conventional fluoran compounds, the fluoran compounds of the general formula I are superior in (1) the solubility in an organic solvent, when used for pressure sensitive record sheets, and (2) the rising for color-development, (3) the low temperature color development and (4) the resistance to self-color development under humidity, when used for heat sensitive record sheets. Thus, they are extremely useful for a color developing record material.

Now, the present invention will be described in detail with reference to the preferred embodiments.

The fluoran compounds of the general formula I include the following specific compounds:

(B) 2-(2-chlorobenzylamino)-6-di-n-butylamino-fluoran of the formula:

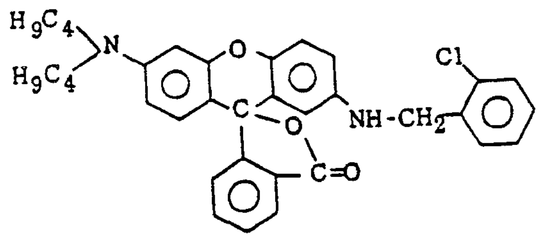

(C) 2-(3-chlorobenzylamino)-6-di-n-butylamino-fluoran of the formula:

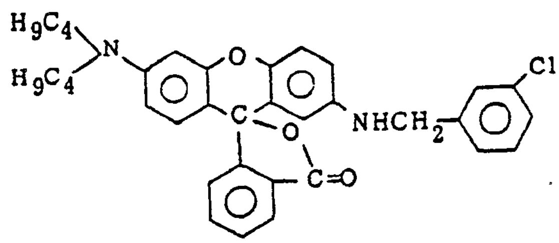

(D) 2-(3-chlorobenzylamino)-6-di-n-butylamino-fluoran of the formula:

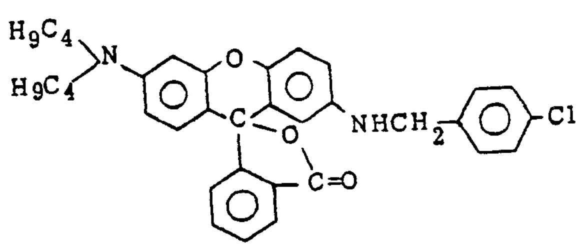

The solubility of the fluoran compounds (B), (C) and (D) of the present invention in organic solvents was compared with that of the above-mentioned conventional fluoran compounds (A—1), (A—2) and (A—3), and the results are shown in Table 1.

TABLE 1
Comparison in the solubility (% by weight) in organic solvents (at 50°C)

| Compounds | | KMC—113 | Hyzole—296 |
|---|---|---|---|
| Conventional compounds | (A—1) | 0.44% | 1.40% |
| | (A—2) | 1.20% | 1.80% |
| | (A—3) | 1.90% | 3.10% |
| Compounds of the present invention | (B) | 3.30% | 4.80% |
| | (C) | 4.20% | 6.30% |
| | (D) | 3.80% | 7.20% |

Note: KMC—113 is an alkyl naphthalene compound manufactured by Kureha Chemical Industry Co. Ltd., and Hyzole—296 is a diallylethane compound manufactured by Nippon Petrochemical Co. Ltd. Both are organic solvents commonly used for pressure sensitive record sheets.

As shown in Table 1, the fluoran compounds (B), (C) and (D) of the present invention are superior to the conventional fluoran compounds (A—1), (A—2) and (A—3) in the solubility in the organic solvents and thus have good oil compatibility which is essential for a color developing material.

The inventors have conducted extensive researches for fluoran compounds to be used for heat sensitive record sheets which have no substantial self-color development, a high developed color density, a low color-development initiation temperature, a high rising for color development and excellent resistance to self-color development under humidity, and have found that the fluoran compounds of the general formula I have excellent properties to satisfy the above requirements.

The fluoran compounds of the general formula I of the present invention may be prepared by the following method (1) or (2).

(1) A benzylamine compound represented by the general formula II:

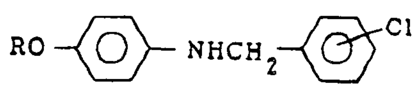

(II)

wherein R is a hydrogen atom or a lower alkyl group, and 2-(2-hydroxy-4-di-n-butylamino)-benzoyl-benzoic acid of the formula:

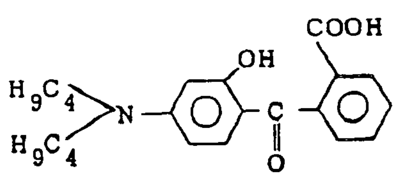

are reacted at a temperature of from 0 to 80°C for from a few hours to some 10 hours in the presence of a condensing agent such as concentrated sulfuric acid. After the reaction, the reaction mixture is poured into water, and an aqueous sodium hydroxide solution is added to bring the pH to from 8 to 10. The precipitate thereby formed, is collected by filtration. To the cake, toluene and an aqueous solution containing from 5 to 10% by weight of sodium hydroxide are added, and the mixture is stirred for 1 to 3 hours under reflux. The toluene layer is separated by liquid phase separation, washed with water and concentrated. The precipitated crystals are collected by filtration. By the filtration of the crystals, a 2-(chloro-substituted benzylamino)-6-di-n-butylamino-fluoran compound of the general formula I is obtainable in high purity and good yield. If necessary, the product is recrystallized from an organic solvent such as methanol, ethanol, toluene or acetone.

(2) 2-Amino-6-di-n-butylamino-fluoran of the formula:

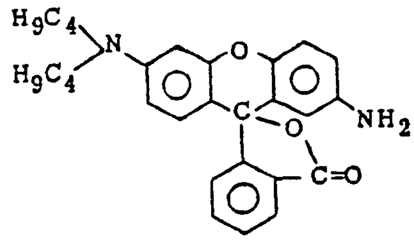

is reacted with a chloro-substituted benzylchloride represented by the general formula III:

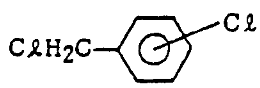

(III)

in water or in a volatile organic inert solvent in the presence of an acid-binding agent such as sodium hydroxide, sodium carbonate or sodium hydrogen carbonate, whereby a fluoran compound of the general formula I is obtained.

From the practical point of view, the method (1) is preferred.

As the condensing agent to be used in the present invention, there may be mentioned concentrated sulfuric acid, acetic acid anhydride, phosphoric acid, polyphosphoric acid, phosphorus oxychloride and zinc chloride. From the practical point of view, it is preferred to use concentrated sulfuric acid.

Now, the present invention will be described in further detail with reference to Examples for the preparation of typical 2-(chloro-substituted benzylamino)-6-di-n-butylamino-fluoran compounds of the present invention and Application Examples.

Example 1

2-(2-Chlorobenzylamino)-6-di-n-butylamino-fluoran [Compound (B)]

To 832 g of 95% sulfuric acid, 93 g of 2-(2-hydroxy-4-di-n-butylamino)-benzoyl-benzoic acid was added and completely dissolved at a temperature of about 20°C, and then 65.5 g of 4-(2-chlorobenzylamino)-anisole was added and reacted therewith at a temperature of from 20 to 30°C for 72 hours. After the reaction, the reaction mixture was poured into 2 liters of ice water and adjusted to a pH of 8.0 by an addition of a 10% sodium hydroxide aqueous solution, whereupon the precipitate was collected by filtration. To the cake thus obtained, 3 liters of toluene and 1.5 liters of a 10% sodium hydroxide aqueous solution were added, and the mixture was stirred for 2 hours under reflux. Then, the toluene layer was separated by liquid separation, washed with water and concentrated. The precipitated crystals were collected by filtration. The crystals were dried to obtain slightly pink 2-(2-chlorobenzylamino)-6-di-n-butylaminofluoran. This fluoran compound was recrystallized from methanol, whereby 100 g of crystals having a melting point of from 150 to 152°C were obtained. This compound had $\lambda_{max}$ at 431 nm (molecular extinction coefficient "MEC": $1.75 \times 10^4$), at 452 nm (MEC: $1.67 \times 10^4$) and at 595 nm (MEC: $2.05 \times 10^4$) as measured in 95% acetic acid. A solution of this compound in toluene was colorless. When brought in contact with silica gel, the compound readily underwent color-development and turned greenish black.

The 4-(2-chlorobenzylamino)-anisole used as the starting material in Example 1, was prepared in the following manner.

Into 2.8 liters of methanol, 292 g of p-anisidine and 350 g of 2-chlorobenzaldehyde were dissolved and stirrer for 2 hours under reflux. Then, the reaction mixture was cooled to 30°C, and after an addition of 2.5 liters of methanol, 134 g of sodium borohydride was gradually added in 1 hour, whereby the formed Schiff base was reduced. After the addition, the reaction mixture was stirred for from 1 to 2 hours and then poured into 20 liters of water. The precipitated white crystals were dried to obtain 590 g of 4-(2-chlorobenzylamino)-anisole. This product had a melting point of from 53 to 54°C.

Example 2

2-(3-Chlorobenzylamino)-6-di-n-butylamino-fluoran [Compound (C)]

In 832 g of 95% sulfuric acid, 2-(2-hydroxy-4-di-n-butylamino)-benzoyl-benzoic acid and 65.5 g of 4-(3-chlorobenzylamino)-anisole were reacted and treated in the same manner as in Example 1, whereby slightly pink crude crystals having a melting point of from 148 to 152°C were obtained. The crude crystals were washed with ethanol, whereby 98 g of substantially colorless 2-(3-chlorobenzylamino)-6-di-n-butylamino-fluoran having a melting point of from 151 to 152.5°C was obtained. This compound had $\lambda_{max}$ at 432 nm (MEC: $1.67 \times 10^4$), at 454 nm (MEC: $1.59 \times 10^4$) and at 597 nm (MEC: $1.99 \times 10^4$) as measured in

95% acetic acid. A solution of this compound in toluene was colorless. When brought in contact with silica gel, the compound readily underwent color-development and turned greenish black.

Example 3

2-(4-Chlorobenzylamino)-6-di-n-butylamino-fluoran [Compound (D)]

In 832 g of 95% sulfuric acid, 93 g of 2-(2-hydroxy-4-di-n-butylamino)-benzoyl-benzoic acid and 65.5 g of 4-(4-chlorobenzylamino)-anisole were reacted and treated in the same manner as in Example 1, whereby slightly pink crude crystals having a melting point of from 130 to 133°C were obtained. The crude crystals were washed with methanol, whereby 101 g of substantially colorless 2-(4-chlorobenzylamino)-6-di-n-butylamino-fluoran having a melting point of from 141 to 143°C was obtained. This compound had $\lambda_{max}$ at 432 nm (MEC: $1.64 \times 10^4$), at 453 nm (MEC: $1.56 \times 10^4$) and at 596 nm (MEC: $1.92 \times 10^4$) as measured in 95% acetic acid. A solution of this compound in toluene was colorless. When brought in contact with silica gel, this compound readily underwent color-development and turned greenish black.

Application Example 1

To 2.0 g of Compound (B) prepared in Example 1, 20 g of water and 20 g of an aqueous solution containing 10% by weight of polyvinyl alcohol were added. The mixture was dispersed and mixed in a ball mill at room temperature for 24 hours to obtain a slurry wherein the particle size of the compound was about 3 µm.

On the other hand, 7 g of bisphenol A was added to 10 g of water and 40 g of an aqueous solution containing 10% by weight of polyvinyl alcohol. The mixture was dispersed and mixed in a ball mill at room temperature for 24 hours to obtain a slurry. The solid substance in this slurry had an average particle size of about 5 µm.

Both slurries were mixed, and the mixture was uniformly dispersed and mixed at room temperature for 1 hour, whereby a slurry mixture was prepared. This slurry mixture was coated on one surface of a normal paper of 50 g/m$^2$ by means of a wire bar coater (wound wire: 0.35 mm in diameter) in an amount of the coated compound being 1.5 g/m$^2$ of the paper. The coated paper was dried in air at room temperature, whereby a heat sensitive record sheet having a substantially colorless heat sensitive layer was obtained. This heat sensitive record sheet will be referred to as No. 1.

In the same manner, heat sensitive record sheets No. 2 and No. 3 were prepared by using Compounds (C) and (D).

Further, for the purpose of comparison, heat sensitive record sheets No. 4 to No. 8 were prepared in the same manner as above by using known 2-anilino-3-methyl-6-diethylamino-fluoran [Compound (E)], 2-anilino-3-methyl-6-N-methyl-N-cyclohexylamino-fluoran [Compound (F)], 2-benzylamino-6-diethylamino-fluoran [Compound (A—1)], 2-(2-chlorobenzylamino)-6-diethylamino-fluoran [Compound (A—2)] and 2-(4-chlorobenzylamino)-6-diethylamino-fluoran [Compound (A—3)].

These heat sensitive record sheets were subjected to the following tests.

(1) Color-development performance test

Heat sensitive record sheets No. 1 to No. 3 and comparative sheets No. 4 to No. 8 were heated at a temperature of 150°C for 5 seconds, whereby the developed color hue, the developed color density and the initial color density were measured by means of Macbeth reflex densitometer RD—514 model with a visual filter (Wratten #106).

(2) Color-development characteristic test

Heat sensitive record sheets No. 1 to No. 3 and comparative sheets No. 4 to No. 8 were heated for color-development within the temperature range of from 70 to 160°C for 5 seconds, whereby the color density at each temperature was measured in the same manner as in the above test (1), and the color-development initiation temperature and the rising for the color-development were calculated from the relationship between the temperature and the color density.

(3) The resistance to self-color development under humidity

Heat sensitive record sheets No. 1 to No. 3 and Comparative sheets No. 4 to No. 8 were kept at 50°C for 24 hours under a relative humidity (RH) of 90%, and the color density was measured by means of Macbeth reflex densitometer RD—514 model with a visual filter (Wratten #106).

The results of the above-mentioned color-development performance test (1), the results of the measurement of the color-development values (2) and the results of the measurement of the resistance to self-color development under humidity (3) are shown in the following Table.

TABLE 2
Color-development performance and color-development characteristic values of heat sensitive record sheets

| | Heat sensitive record sheet | | Color-development performance | | Color-development characteristic values | | Resistance to self-color development under humidity | |
|---|---|---|---|---|---|---|---|---|
| | Compound No. | Sheet No. | Developed color hue | Developed color density | Color-development initiation temperature (°C) | Rising for color develop-ment | Initial color density | Self-developed color density |
| Present invention | B | 1 | Greenish black | 1.15 | 92 | 4.4 | 0.07 | 0.25 |
| | C | 2 | Greenish black | 1.16 | 91 | 4.6 | 0.08 | 0.27 |
| | D | 3 | Greenish black | 1.18 | 88 | 5.3 | 0.08 | 0.28 |
| Com-parative Examples | E | 4 | Reddish black | 1.12 | 85 | 1.2 | 0.12 | 0.50 |
| | F | 5 | Reddish black | 1.10 | 92 | 2.4 | 0.08 | 0.36 |
| | A—1 | 6 | Greenish black | 1.12 | 96 | 4.1 | 0.14 | 1.10 |
| | A—2 | 7 | Greenish black | 1.13 | 96 | 4.2 | 0.13 | 0.88 |
| | A—3 | 8 | Greenish black | 1.12 | 95 | 4.3 | 0.14 | 1.04 |

Note: The color development by heating was conducted by means of rhodiaceta model thermotest rhodiaceta (manufactured by French National Fiber Research Institute) at a heating temperature of from 70 to 160°C for a heating time of 5 seconds under a load of 100 $g/cm^2$.

It is evident from the results shown in the above Table that the heat sensitive record sheets wherein the fluoran compounds of the present invention are used, are far superior in the color-development performance, the developed color characteristics and the resistance to self-color development under humidity to the comparative heat sensitive record sheets wherein comparative fluoran compounds are used. Thus, the industrial value for practical application of the present invention is considerably high.

**Claims**

1. A 2-(chloro-substituted benzylamino)-6-di-n-butylamino-fluoran compound represented by the general formula I:

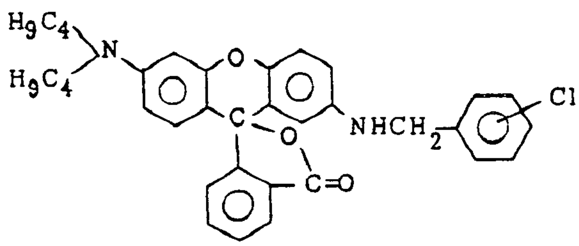

(I)

2. The fluoran compound according to Claim 1, which is 2-(2-chlorobenzylamino)-6-di-n-butylamino-fluoran.
3. The fluoran compound according to Claim 1, which is 2-(3-chlorobenzylamino)-6-di-n-butylamino-fluoran.
4. The fluoran compound according to Claim 1, which is 2-(4-chlorobenzylamino)-6-di-n-butylamino-fluoran.

**Patentansprüche**

1. Eine 2-(Chlor-substituiertes Benzylamino)-6-di-n-butylamino-fluoranverbindung gemäß der allgemeinen Formel I

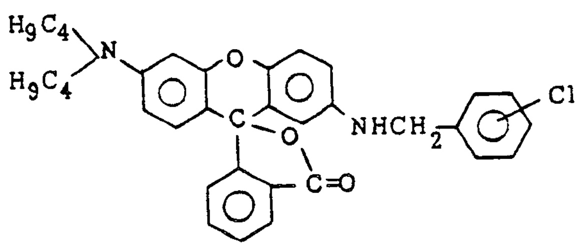

(I)

2. Die Fluoranverbindung gemäß Anspruch 1, nämlich 2-(2-Chlorbenzylamino)-6-di-n-butylamino-fluoran.
3. Die Fluoranverbindung gemäß Anspruch 1, nämlich 2-(3-Chlorbenzylamino)-6-di-n-butylamino-fluoran.
4. Die Fluoranverbindung gemäß Anspruch 1, nämlich 2-(4-Chlorbenzylamino)-6-di-n-butylamino-fluoran.

**Revendications**

1. Dérivé 2-(benzylamino chloro-substitué)-6-di-n-butylamino-fluoranne, représenté par la formule générale I:

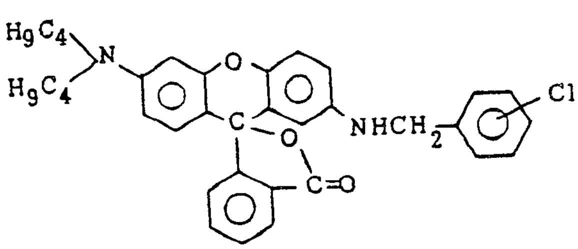

(I)

2. Dérivé du fluoranne selon la revendication 1 qui est le 2-(2-chlorobenzylamino)-6-di-n-butylamino-fluoranne.

3. Dérivé du fluoranne selon la revendication 1, qui est le 2-(3-chlorobenzylamino)-6-di-n-butylamino-fluoranne.

4. Dérivé du fluoranne selon la revendication 1, qui est le 2-(4-chlorobenzylamino)-6-di-n-butylamino-fluoranne.